# EUROPÄISCHE PATENTSCHRIFT

(11) **EP 0 677 282 B1**
(45) Veröffentlichungstag und Bekanntmachung des Hinweises auf die Patenterteilung: **03.03.1999**
(21) Anmeldenummer: 95104632.5
(22) Anmeldetag: 29.03.1995
(51) Int. Cl.: A61F 5/01

(54) **Knieschiene**
Knee splint
Eclisse de genou

(30) Priorität: 01.04.1994 DE 4411469
(43) Veröffentlichungstag der Anmeldung: 18.10.1995
(73) Patentinhaber: Beiersdorf Aktiengesellschaft, 20245 Hamburg (DE)
(72) Erfinder: Herzberg, Thorsten, D-21149 Hamburg (DE)
(74) Vertreter: Richter, Werdermann & Gerbaulet

(56) Entgegenhaltungen:
- WO-A-89/04155
- FR-A- 2 566 850
- US-A- 4 090 508
- US-A- 4 681 097

## Beschreibung

Die Erfindung betrifft eine Knieschiene mit einer am Bein anzulegenden, auf der Vorderseite des Beines mittels Verschlußbänder zusammengehaltenen Hülse aus einem abgepolsterten Rahmen.

Operative Eingriffe am Knie verlangen eine postoperative Ruhigstellung. Hierfür ist durch die FR-A-2 566 850 eine Knieschiene mit einer am Bein anzulegenden, auf der Vorderseite des Beines mittels Verschlußbänder zusammengehaltenen Hülse aus einem abgepolsterten Rahmenteil bekannt, wobei die Knieschiene aus einem metallischen Rahmen aus einem oberen, am Oberschenkel des Beines dorsal anlegbaren kürzeren, halbschalenförmigen Rahmenteil und einem unteren, am Unterschenkel des Beines dorsal anlegbaren längeren, halbschalenförmigen Rahmenteil besteht, wobei die beiden Rahmenteile miteinander verbunden sind und jeder Rahmenteil von einem U-förmigen Stabprofil mit einem etwa halbkreisförmig verlaufenden Steg zur rückwärtigen Anlage an den Oberschenkel bzw. den Unterschenkel des Beines gebildet ist, und wobei die beiden Rahmenteile derart miteinander verbunden sind, daß deren beiden U-förmigen Stabprofile einander zugekehrt sind. Diese Knieschiene ist nicht mit in Knieschienenlängsrichtung verlaufenden, verformbaren und federnden Fixierungsstäben versehen, um eine flexible, instabile Schale mit Nachformungsmöglichkeiten zu erhalten.

Durch die DE-A-31 23 148 ist eine Knieschiene mit einem flexiblen Mantel bekannt, dessen Seitenränder nach dem Anlegen des Mantels an das Bein auf der Vorderseite des Beins zusammengehalten werden und je eine Aussparung für die Kniescheibe aufweisen, wobei längs dieser Seitenränder verstellbare Verschlußelemente vorgesehen sind und wobei der Mantel an den Bereichen, die nach dem Anlehen des Mantels an das Bein an den Seitenflächen und der Rückseite des Beines zu liegen kommen, mit Versteifungsstäben versehen ist. Der Mantel dieser Knieschiene ist aus mehreren anatomiegerechten Zuschnitten zusammengesetzt, wobei die Zuschnitte und die Versteifungsstäbe eine zur Fixierung des Kniegelenks in einer Beugestellung von etwa 20° bestimmte Form und Krümmung aufweisen.

Auch die Knieschiene nach der DE-A-42 29 044 besteht aus einem flexiblen Mantel, der an seinen Seitenrändern mit Verschlußelementen versehen ist, die den Mantel nach dem Anlegen an das Bein in Form einer geschlossenen Röhre zusammenhalten, wobei die Längsachse des den Unterschenkel umgebenden Teils eine Winkelstellung zur Längsachse des den Oberschenkel umgebenden Teils einnimmt. Bei dieser Knieschiene ist der den Mantel bildende Zuschnitt einteilig ausgebildet und in eine Ebene abwickelbar, wobei in abgewickeltem Zustand eine sich im wesentlichen parallel zur Beinlängsachse erstreckende Ausnehmung vorgesehen ist, welche längs der Beinrichtung größere Abmessungen aufweist als quer dazu. Die Ausnehmung erreicht nicht an mindestens einem ihrer Enden die jeweils nächstliegende Kante des Zuschnitts. Die beiden längsgerichteten Seitenkanten der Ausnehmung sind bei fertiggestellter Knieschiene miteinander verbunden.

In den beiden erstgenannten Fällen bestehen die Knieschienen aus einem flexiblen Mantel. Ist dieser Mantel aus mehreren Zuschnitten zusammengesetzt, so ist stets eine große Genauigkeit bei der Näharbeit erforderlich. Ein ungenaues Aneinanderliegen und Zusammenfügen der einzelnen Zuschnitte kann zu einer fehlerhaften Form und damit zu seinem schlechten Sitz der Schiene führen. Beide bekannten Knieschienen sind so ausgebildet, daß sie das Kniegelenk in einer vorgegebenen Beugestellung von etwa 20° fixieren, wobei eine im Verlauf der Behandlung auftretende Lockerung der Knieschiene korrigierbar ist. Um diese vorgegebene Beugestellung einhalten zu können, sind die Mäntel der Knieschienen aus mehreren anatomiegerechten Zuschnitten zusammengesetzt, wobei die Zuschnitte und die Versteifungsstäbe eine zur Fixierung des Kniegelenkes in einer Beugestellung von etwa 20° bestimmte Form und Krümmung aufweisen. Aufgrund der vorgegebenen, die Knieschienen bildenden Mäntel ergeben sich oftmals Paßformprobleme und Anpassungsschwierigkeiten. Letztlich müssen diese bekannten Knieschienen maßgefertigt sein. Hinzu kommt, daß auch bei stillgelegtem Knie der Patient mit den bekannten Knieschienen keine Streckübungen machen kann. Da die Mäntel dieser Knieschienen im angelegten Zustand das Bein allseitig umhüllen, ist ein Wärmestau nicht vermeidbar.

Durch die US-A-3,831,467 ist eine Knieorthese bekannt, die aus einem elastischen Flächengebilde besteht, das hüllenartig im Kniebereich am Ober- und Unterschenkel angelegt und mittels einer Vielzahl von Verschlußbändern am Bein festgehalten wird. Das Bein wird im angelegten Zustand der Knieorthese von dieser ganz umschlossen; nur eine Öffnung für die Patella wird freigelassen. Zur Versteifung der Orthese, durch die ein Beugen des Knies verhindert werden soll, sind an dem Flächengebilde Versteifungsstäbe vorgesehen, von denen die mittleren drei Versteifungsstäbe gegenüber der Patella, also die direkt hinter der Kniekkehle angeordneten Versteidungsstäbe, die wichtigsten sind. Zusätzlich kann seitlich noch je ein Versteifungsstab vorgesehen sein. Wegen des natürlichen Hohlraumes der Kniekehle liegen die Versteifungsstäbe dort nicht am Bein an, was ein Hin- und Herrutschen der Orthese am Bein bewirken kann, auch wenn die Verschlußbänder fest angezogen sind. Aus diesem Grund ist ein weiches Polster auf der inneren Oberfläche der Knieorthese an dieser Stelle angebracht. Dieses Polster füllt lediglich die Kniekehle aus; es hält jedoch das Knie nicht in der für eine erfolgreiche Behandlung erforderliche Beugehaltung von 10° bis 20°, da das Polster relativ flach ausgebildet ist und lediglich zur Verminderung des Verrutschens der angelegten Orthese beitragen soll.

Aufgabe der vorliegenden Erfindung ist es, eine post-operative oder bei Beinverletzungen anwendbare Knieschiene zur Ruhigstellung des Kniegelenkes in gestreckter Stellung, zweckmäßigerweise in funktionaler Beugestellung, unter Einbeziehung einer Beinbeweglichkeit in einem vorgegebenen Winkelbereich und der Möglichkeit, diesen in Anpassung an den Genesungsverlauf ändern zu können, zu schaffen, die eine Anpassung an die Anatomie des Beines und eine genaue Positionierung des Kniegelenkes ermöglicht, wobei die Knieschiene eine symmetrische Konstruktion aufweisen soll, um diese sowohl für das linke Bein als auch für das rechte Bein einsetzen zu können.

Gelöst wird diese Aufgabe durch die im Anspruch 1 angegebenen Merkmale.

Die die Knieschiene bildende Hülse weist hiernach einen bevorzugterweise metallischen Rahmen aus einem oberen, am Oberschenkel des Beines dorsal anlegbaren, kurzen, halbschalenförmigen Rahmenteil und einem unteren, am Unterschenkel des Beines ebenfalls dorsal anlegbaren längeren, halbschalenförmigen Rahmenteil auf, wobei jeder Rahmenteil von einem U-förmigen Stabprofil mit einem etwa halbkreisförmig verlaufenden Steg zur Anlage an den Oberschenkel bzw. Unterschenkel des Beines gebildet ist, an dem seitlich und benachbart zu den beiden Stabprofilschenkeln eine Anzahl von einendseitig befestigten, in Knieschienenlängsrichtung nach oben und nach unten verlaufenden, verformbaren,federnden und eine hohe Eigensteifigkeit aufweisenden, einarmigen dorsalen Fixierungsstäbe befestigt sind, die der anatomischen Form von Oberschenkel und Unterschenkel des Beines in etwa angepaßt sind, so daß eine flexible, instabile Schale mit Nachformungsmöglichkeiten erhalten wird, die es ermöglicht, die Knieschiene mühelos an die Anatomie des Beines anpassen zu können. Des weiteren ist die Knieschiene im Bereich ihrer beiden Endbereiche mit je einem Verschlußband und mit je einem weiteren, oberhalb und unterhalb des Knies bei angelegter Knieschiene verlaufenden Verschlußband versehen.

Nach einem weiteren Merkmal der Erfindung sind die beiden Rahmenteile des Knieschienenrahmens vermittels Scharniere gelenkig miteinander verbunden, wobei die Scharniere so ausgebildet sind, daß die beiden Rahmenteile des Knieschienenrahmens bei angelegter Knieschiene in einer vorgegebenen Winkelstellung arretierbar sind oder auf einen Winkelbereich mit einer Winkelstellung für eine Flexion oder Extension des Beines einstellbar sind, der eine definierte Bewegung des Beines zuläßt, wobei dieser Winkel- und/oder Bewegungsbereich des Beines dem Genesungsfortschritt veränderbar und anpaßbar ist. Je nach fortschreitender Genesung des Beines ist somit die Möglichkeit für eine entsprechende Beinbewegung und Beugung in vorgegebenem Winkelbereich gegeben. Der Winkelbereich wird in Anpassung an den jeweiligen Zustand des Beines eingestellt und verändert, so daß eine definierte Beinbewegung erhalten wird.

Zur Erhöhung des Tragekomforts sind die Rahmenteile des Knieschienenrahmens abgepolstert.

Dadurch, daß die Verschlußbänder für die Halterung der Knieschiene am Bein an dem Knieschienenrahmen an bestimmten Stellen positioniert sind, nämlich im Bereich der Enden der Knieschiene und dicht oberhalb und unterhalb des Knies, ist eine genaue Positionierung des Kniegelenkes möglich. Wesentlich ist dabei, daß die Gelenkachsen der die beiden Rahmenteile miteinander verbindenden Gelenke miteinander kongruieren, d.h. deckungsgleich sind. Mit dieser Knieschiene wird das Bein im Kniebereich fest positioniert und gut gehalten. Trotz der gewissen Flexibilität, die die beiden Halbschalen der Knieschiene aufweisen, ist eine hohe Stabilität gegeben. Die Verwendung von dorsalen Fixierungsstäben ermöglicht ein müheloses Anpassen der Knieschiene an die Anatomie des Beines. Die Flexibilität dieser Fixierungsstäbe ermöglicht auch ein späteres Nachformen und Anpassen der Knieschiene, was dadurch möglich ist, daß die Fixierungsstäbe dorsal oberhalb und unterhalb des Knies an dem Knieschienenrahmen befestigt sind.

Hinzu kommt, daß die Knieschiene mit einer geringen Anzahl von Verschluß- und Haltebändern auskommt. Bevorzugterweise erfolgt die Befestigung der Knieschiene an vier Stellen des Beines, wobei ein unverrückbarer Sitz der angelegten Knieschiene insbesondere dadurch erreicht wird, daß die Knieschiene mittels zweier Verschlußbänder am Bein gehalten wird, die oberhalb und unterhalb des Knies des Beines verlaufen.

Die Abpolsterung des Rahmens der Knieschiene erfolgt mittels zweier auf die Rahmenteile aufschiebbarer Polsterzuschnitte, die rückseitig mit einer der Anzahl der Fixierungsstäbe der Rahmenteile entsprechenden Anzahl von Taschen mit Einschuböffnungen versehen sind, in die die Fixierungsstäbe der beiden Rahmenteile des Knieschienenrahmens eingeschoben werden, wobei der obere Polsterzuschnitt auf die Fixierungsstäbe des oberen Rahmenteils und der untere Polsterzuschnitt von unten auf die Fixierungsstäbe des unteren Rahmenteils aufgeschoben werden. Die Befestigung und Halterung der Polsterzuschnitte erfolgt mittels geeigneter Verbindungsmittel, wie z.B. Druckknöpfe, Klettverschlußverbindungen, wobei auch andersartig ausgebildete Verschlußmittel eingesetzt werden können.

Hinzu kommt, daß die unverrückbare Halterung der am Bein angelegten Knieschiene insbesondere durch die Anordnung der vier Verschlußbänder erreicht wird, von denen jeweils das obere und das untere Verschlußband an den Polsterzuschnitten vorgesehen ist, während die im Kniebereich, d.h. oberhalb und unterhalb des Knies verlaufenden Verschlußbänder an den beiden Rahmenteilen des Knieschienenrahmens befestigt sind. Auf diese Weise wird der Knieschienenrahmen selbst direkt am Bein gehalten und nicht ausschließlich über die Polsterzuschnitte.

Zur Druckentlastung der Patella ist an dem oberhalb des Knies verlaufenden Verschlußband ein Kissen bzw. Druckpolster angeordnet, das aufgrund seiner Formgebung eine Anpassung an die Patella ermöglicht. Auf diese Weise wird bei angelegter Knieschiene die Patella entlastet. Auch an dem unterhalb des Knies verlaufenden Verschlußband kann ein Kissen bzw. Druckpolster vorgesehen sein.

Die Knieschiene besteht somit aus einer Rahmengrundkonstruktion mit zwei halbschalenförmigen und stabilen, abgepolsterten Rahmenteilen, die in ihren Endbereichen Verschlußbänder tragen. Durch diese Konstruktion wird eine flexible, jedoch stabile Halbschale erhalten, in die das Bein gelegt werden kann. Nach Anbringen der Verschlüsse wird eine das Bein umgebende Hülse geschaffen. Um das Knie in Beugestellung zu halten oder um einen definierten Bewegungsbereich für das Bein zu schaffen, werden die beiden Rahmenteile des Knieschienenrahmens mit ihren Polsterungen in eine vorgegebene Winkelstellung gebracht oder auf einen vorgegebenen Winkelbereich eingestellt, wobei die die beiden Rahmenteile miteinander verbindenden Gelenke dann in der vorgegebenen Winkelstellung arretiert oder auf den gewünschten Beinbewegungsbereich eingestellt werden.

Mit der erfindungsgemäß ausgebildeten Knieschiene werden noch weitere Vorteile erreicht:
- Eine sehr offene Konstruktion ohne Wärmestau.
- Eine gute Stabilisierung des Knies.
- Einfache Handhabung durch bevorzugterweise nur vier Verschlüsse.
- Gute Paßform, geringes Gewicht und Anpassungsfähigkeit an die Anatomie des jeweiligen Beines, an das die Knieschiene angelegt werden soll.
- Die Knieschiene kann problemlos am liegenden Patienten geöffnet werden; die Winkelstellung bzw. der Bewegungsspielraum der beiden Rahmenteile des Knieschienenrahmens ist jederzeit mühelos veränderbar, um den Beugebereich des Beines dem jeweiligen Genesungszustand anpassen zu können, so daß eine Gelenkführung und Beinbewegung in Flexion und Extension möglich ist.
- Durch die bevorzugterweise rohrförmigen Fixierungsstäbe an den beiden Rahmenteilen des Knieschienenrahmens formt sich die Knieschiene dem Bein besser an.
- Die Fixierungsstäbe dienen zur Stabilisierung des Beines und können einfach abgebogen werden, um die Knieschiene, falls erforderlich, genau anzupassen.
- Die Operationswunden werden nicht von einer Bandage abgedeckt.

Als Knieschiene bietet diese eine hohe Therapiesicherheit. Das Knie ist immobilisierbar und definiert zu stabilisieren. Ein anhaltender, gleichbleibender Stabilisierungseffekt wird erreicht. Das Knie wird in einer definierten, leichten Beugestellung ruhiggestellt, die das vordere Kreuzband entspannt. Die Knieschiene sitzt gut am Bein an und ist restabil verankert. Eine gute Weichteilabstützung wird sowohl am Oberschenkel als auch am Unterschenkel erreicht. Die Knieschiene läßt sich unterschiedlichen Ober- und Unterschenkelweiten anpassen.

Mit der Knieschiene wird ferner ein hoher Tragekomfort erhalten, denn die Knieschiene scheuert nicht, schnürt nicht ein, trägt wenig auf, ist sehr leicht aufgrund des geringen Gewichtes; sie ist hautfreundlich, luft- und wasserdampfdurchlässig und hat keine unangenehme Wärmewirkung. Die Knieschiene läßt sich einfach anlegen. Der Heilungsprozeß ist gut kontrollierbar. Eine postoperative Wundversorgung ist möglich. Durch Verwendung geeigneter Materialien ist die Knieschiene schmutzunempfindlich. Durch die Verwendung gesonderter Polsterzuschnitte, die auf die Rahmenteile des Knieschienenrahmens aufgezogen sind, sind die Polster mühelos abnehmbar, reinigbar und desinfizierbar.

Weiterentwicklungen der Erfindung sind Gegenstand der Unteransprüche.

Ausführungsbeispiele der Erfindung sind in den Zeichnungen dargestellt. Es zeigt
Fig. 1 in einer schaubildlichen Ansicht eine an Ober- und Unterschenkel angelegte Knieschiene aus zwei halbschalenförmig ausgebildeten, gepolsterten Orthesenteilen,
Fig. 2 in einer schaubildlichen Ansicht die Knieschiene gemäß Fig.1,
Fig. 3 in einer Ansicht von oben die Knieschiene,
Fig. 4 in einer Seitenansicht die Knieschiene mit gestreckten Rahmenteilen,
Fig. 5 eine schaubildliche Vorderansicht des aus zwei miteinander gelenkig verbundenen Rahmenteilen bestehenden Knieschienenrahmens,
Fig. 6 eine schaubildliche Rückansicht des Knieschienenrahmens,
Fig. 7 eine Ansicht auf die gepolsterte Vorderseite des Polsterzuschnitts für den oberen Rahmenteil des Knieschienenrahmens,
Fig. 8 in einer Rückansicht den oberen Rahmenteil des Knieschienenrahmens gemäß Fig.7,
Fig. 9 eine Ansicht der gepolsterten Vorderseite des Polsterzuschnitts für den unteren Rahmenteil des Knieschienenrahmens,
Fig.10 in einer Rückansicht den unteren Rahmenteil des Knieschienenrahmens gemäß Fig.9,
Fig.11 in einer schaubildlichen Vorderansicht ein auf das untere Verschlußband des oberen Rahmenteils des Knieschienenrahmens aufgeschobenes Polster mit ausgeformter Ausnehmung für die Patella und
Fig.12 in einer Rückansicht das an dem unteren Verschlußband des oberen Rahmenteils des Knieschienenrahmens verschieblich befestigte Polster gemäß Fig.11.

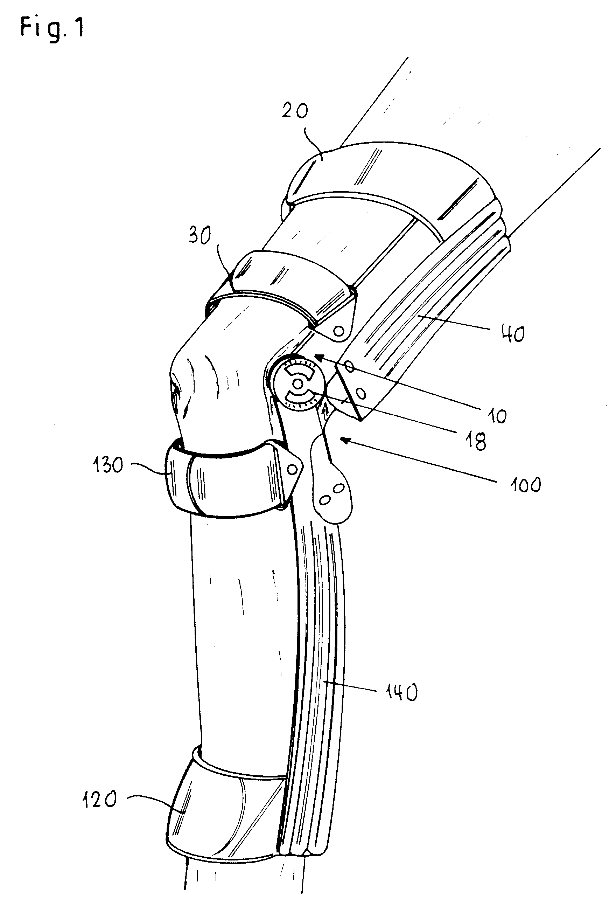

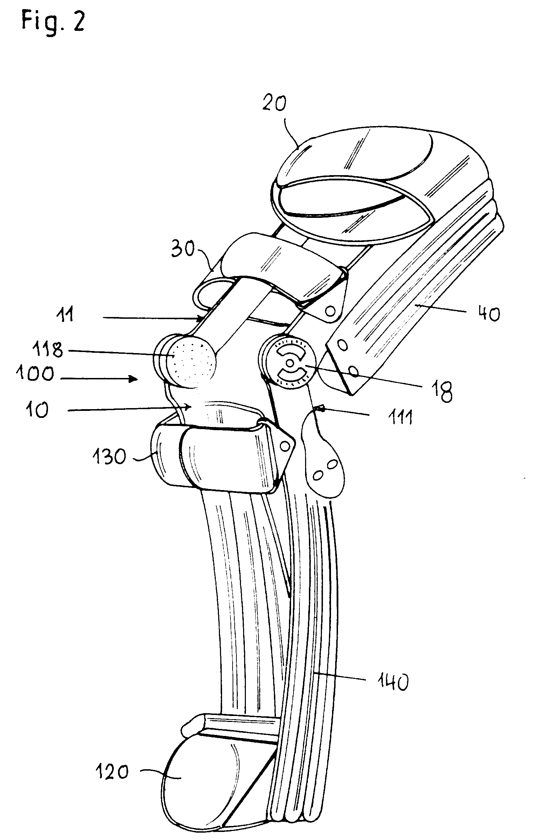

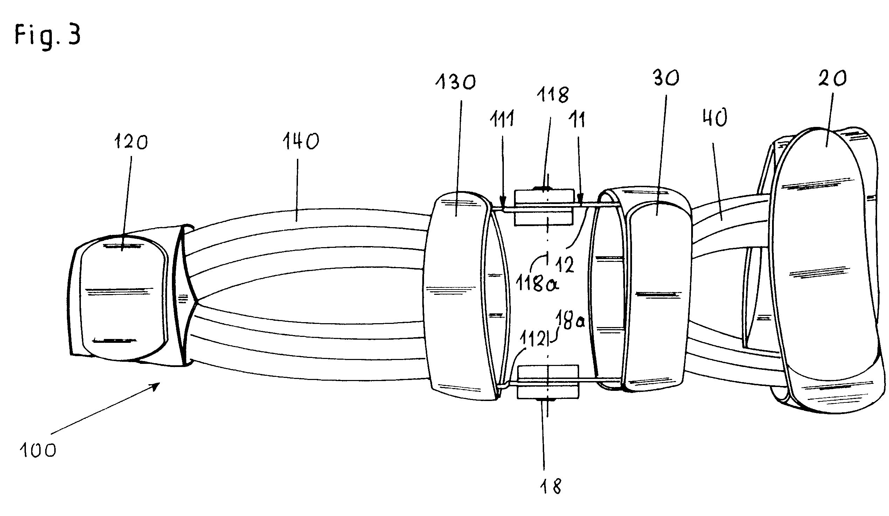

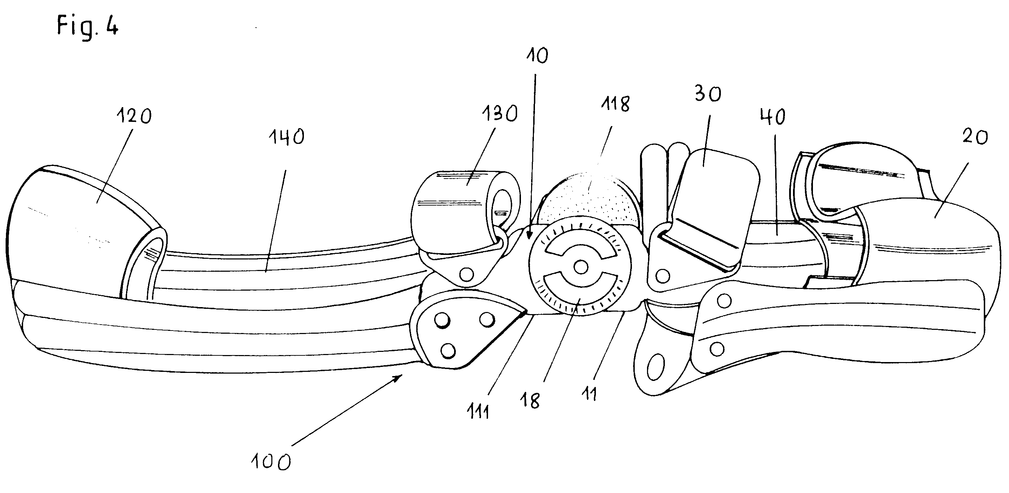

Gemäß Fig.1 bis 4 besteht die Knieschiene 100 aus einer am Bein eines Patienten anzulegenden und auf der Vorderseite des Beines zusammengehaltenen Hülse, die von einem gepolsterten Rahmen 10 mit Verschlußbändern 20,120 und 30,130 gebildet wird.

Die Knieschiene 100 besteht aus einem bevorzugterweise metallischen Rahmen 10 aus einem oberen, am Oberschenkel des Beines dorsal anlegbaren kürzeren, halbschalenförmigen Rahmenteil 11 und einem unteren, am Unterschenkel des Beines ebenfalls dorsal anlegbaren, längeren, halbschalenförmigen Rahmenteil 111. Jeder Rahmenteil 11, 111 ist gebildet von einem U-förmigen Stabprofil 12 bzw. 112 mit einem etwa halbkreisförmig verlaufenden Steg 13 bzw. 113 zur Anlage an den Oberschenkel bzw. Unterschenkel des Beines. An dem Steg 13 bzw. 113 eines jeden Rahmenteils 11,111 des Knieschienenrahmens 10 ist seitlich und benachbart zu den beiden Stabprofilschenkeln 14,15 bzw. 114,115 eine Anzahl von einendseitig befestigten, in Knieschienenlängsrichtung nach oben und nach unten verlaufenden, verformbaren, federnden und eine hohe Eigensteifigkeit aufweisenden, einarmigen,dorsalen Fixierungsstäben befestigt, die der anatomischen Form von Oberschenkel und Unterschenkel des Beines angepaßt sind. Der Rahmen 10 kann auch aus anderen Materialien, wie z.B. aus Kunststoff oder einem Kohlefaser-Material, bestehen.

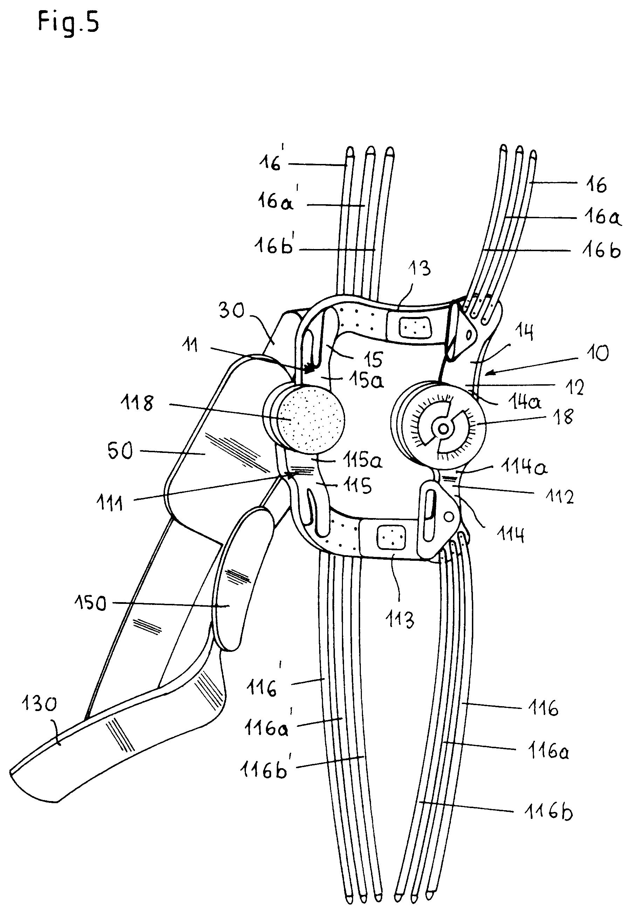

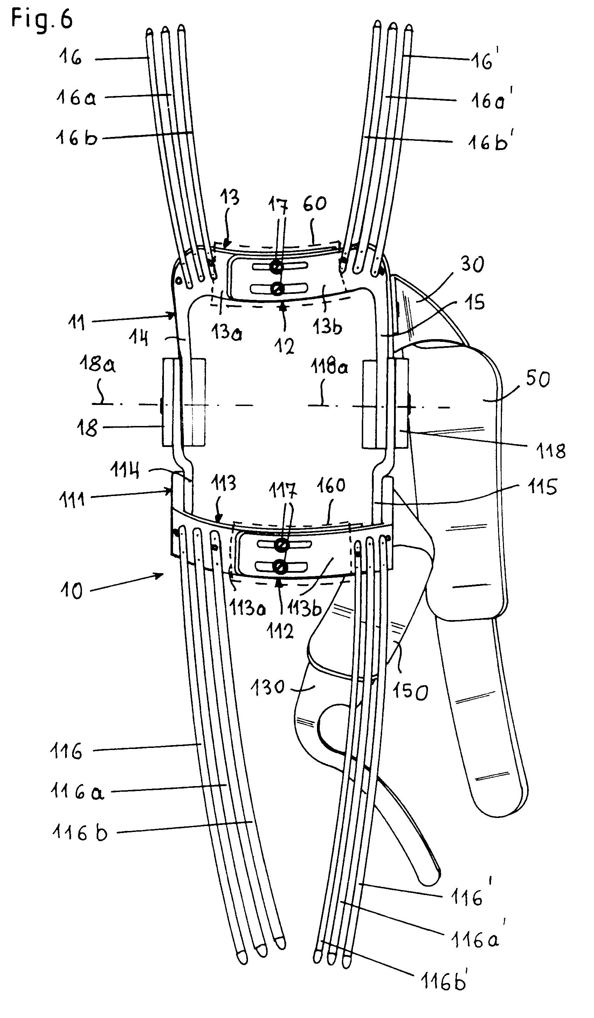

Bei dem in Fig.5 und 6 gezeigten Ausführungsbeispiel eines Rahmens 10 für die Knieschiene 100 sind an dem oberen Rahmenteil 11 sechs Fixierungsstäbe 16,16a,16b und 16', 16a', 16b' und an dem unteren Rahmenteil 111 ebenfalls sechs Fixierungsstäbe 116,116a,116b und 116', 116'a,116b' befestigt. Dabei sind jeweils drei Fixierungsstäbe 16,16a,16b und 16',16a',16b' und 116,116a, 116b und 116',116a',116b' zusammengefaßt, wobei die so zusammengefaßten Fixierungsstäbe eines jeden Rahmenteils 11,111 im dorsalen Bereich des Oberschenkels und des Unterschenkels derart angeordnet sind, daß halbschalenförmig ausgebildete Rahmenteile 11,111 gebildet werden, die den Oberschenkel und den Unterschenkel bei angelegter Knieschiene 100 abschnittsweise übergreifen.

Die Anzahl der Fixierungsstäbe des oberen Rahmenteils 11 und des unteren Rahmenteils 111 kann beliebig gewählt sein. Die Anzahl der Fixierungsstäbe ist nicht beschränkt auf die voranstehend beschriebene und dargestellte Anzahl von Stäben. Wesentlich ist, daß die Anordnung der Fixierungsstäbe derart ist, daß halbschalenförmige Gebilde entstehen, die den Oberschenkel und den Unterschenkel des Beines bei angelegter Knieschiene 100 aufnehmen. Diese Rahmenteile 11,111 des Knieschienenrahmens 100 bilden zusammen mit den Verschlußbändern 20,120 und 30, 130 eine Hülse in Form einer flexiblen, jedoch stabilen Halbschale aus zwei stabilen, eine hohe Eigensteifigkeit aufweisenden, den Beinkonturen angepaßten und anpaßbaren Halbschalenteilen.

Die Fixierungsstäbe an den Rahmenteilen 11,111 sind rohrförmig ausgebildet und bestehen beispielsweise aus Aluminium oder einem anderen geeigneten Material, das neben einer hohen Eigensteifigkeit eine gewisse Flexibilität aufweist, um die Fixierungsstäbe den Beinkonturen anpassen zu können. Diese rohrförmigen Fixierungsstäbe können auch aus einem anderen Leichtmetall als Aluminium oder aus einem glasfaserverstärkten Kunststoff bestehen. Auch eine Ausbildung der rohrförmigen Fixierungsstäbe als Kohlefaserstäbe ist möglich. Wesentlich ist, daß die rohrförmigen Fixierungsstäbe kein hohes Gewicht aufweisen.

Auch vollwandig ausgebildete rohrförmige Fixierungsstäbe können eingesetzt werden. Der Querschnitt der eingesetzten Fixierungsstäbe kann kreisförmig, oval sein oder eine andere geometrische Querschnittsform aufweisen. Die bei der Knieschiene 100 eingesetzten Fixierungsstäbe sind trotz Beibehaltung einer hohen Eigensteifigkeit in einem gewissen Grade verformbar, um die Knieschiene den Konturen des Beines anpassen zu können.

Die beiden Rahmenteile 11,111 können starr miteinander verbunden sein, so daß ein einteiliger Rahmen 10 für die Knieschiene 100 erhalten wird. Eine derart ausgebildete Knieschiene 100 ist nur bei gestreckter Beinlage einsetzbar.

Um jedoch das Bein in einer vorgegebenen Beugestellung oder für eine Bewegung in einem definierten Bereich in der Knieschiene 100 halten zu können, ist diese nach einer weiteren Ausführungsform der Erfindung in der Weise ausgebildet, daß die beiden Rahmenteile 11,111 des Knieschienenrahmens 10 über die freien Enden 14a,15a und 114a, 115a der Schenkel 14,15 und 114,115 der beiden U-förmigen Stabprofile 12 und 112 gelenkig miteinander verbunden sind, so daß die beiden Rahmenteile 11,111 in jeweils erforderlichen Winkeln zueinander eingestellt werden können. Beide Rahmenteile 11,111 sind über Gelenkverbindungen 18,118 miteinander verbunden, die mit in an sich bekannter Weise ausgebildeten Feststelleinrichtungen in Wirkverbindung stehen, so daß die jeweils eingestellten Winkelstellungen feststellbar sind. So können beispielsweise Feststelleinrichtungen für die Gelenkverbindungen 18,118 zur Anwendung gelangen, wie diese in US-A-4,982,732 beschrieben sind, der eine Knieschiene mit einer am Bein anzulegenden, auf der Vorderseite des Beines mittels Verschlußbänder zusammengehaltenen Hülse aus zwei Rahmenteilen, die über in den jeweiligen Winkelstellungen der beiden Rahmenteile zueinander feststellbare oder auf Winkelbereiche einstellbare Scharniere gelenkig miteinander verbunden sind, die jedoch aufgrund ihrer konstruktiven Ausgestaltung ein Verrutschen der Knieschiene beim Tragen nicht verhindert, zu entnehmen ist.

Die Anordnung der die beiden Rahmenteile 11,111 miteinander verbindenden Gelenke 18,118 sind derart zueinander angeordnet, daß deren Schwenkachsen 18a,118a zueinander kongruent liegend sind, d.h. deckungsgleich sind, und in einer Achsebene liegen (Fig.5 und 6).

Zur Halterung der an einem Patientenbein angelegten Knieschiene 100 sind die Verschlußbänder 20,30 und 120,130 vorgesehen. Von diesen Verschlußbändern, auf die nachstehend noch näher eingegangen wird, sind die Verschlußbänder 20,120 an den beidseitigen Enden der Knieschiene 100 vorgesehen, während die beiden anderen Verschlußbänder 30,130 im Kniebereich liegend derart angeordnet sind, daß das Verschlußband 30 in einem geringen Abstand oberhalb des Knies und das Verschlußband 130 in einem geringen Abstand unterhalb des Knies angeordnet sind.

Um die beiden Rahmenteile 11,111 des Knieschienenrahmens 10 unterschiedlichen Formen und Größen des Beins anpassen zu können, sind die Weiten der von den beiden Rahmenteilen 11,111 gebildeten Halbschalen veränderbar. Hierzu ist der halbkreisförmig verlaufende Steg 13 bzw. 113 der beiden U-förmigen Stabprofile 12 und 112 eines jeden Rahmenteils 11,111 aus zwei längenveränderbaren Stababschnitten 13a,13b und 113a,113b gefertigt, wobei je zwei Stababschnitte eines jeden Stabprofils 12 bzw. 112 vermittels einer Feststelleinrichtung 17 bzw. 117 in der jeweils eingestellten Weite feststellbar sind. Diese Feststelleinrichtung 17 bzw. 117 kann als Schraubverbindungen ausgebildet sein, wobei es wesentlich ist, daß ein selbsttätiges Lockern der Feststelleinrichtung nicht eintreten kann.

Die U-förmigen Stabprofile 12,112, die Bestandteil der beiden Rahmenteile 11,111 sind, bestehen bevorzugterweise aus einem Flach- bzw. Bandprofil, wobei die die halbkreisförmig ausgebildeten Stege 13,113 und die Stabprofilschenkel 14,15 und 114,115 senkrecht und um 90° zueinander versetzt angeordnet sind, so daß die Stege 13,113 eine Anlagefläche für den Oberschenkel bzw. den Unterschenkel des Beins bilden, wohingegen die seitlichen Schenkel 14,15 und 114,115 der beiden Stabprofile 12,112 seitlich des Beins verlaufend sind (Fig.6).

Um eine gute Anpassung des unteren Rahmenteils 111 des Knieschienenrahmens 10 am Unterschenkel des Beines zu erreichen, sind die Fixierungsstäbe 116,116a,116b und 116',116a',116b' des unteren Rahmenteils 111 bevorzugterweise geradeverlaufend mit Abwinkelungen im unteren Wadenbereich ausgebildet.

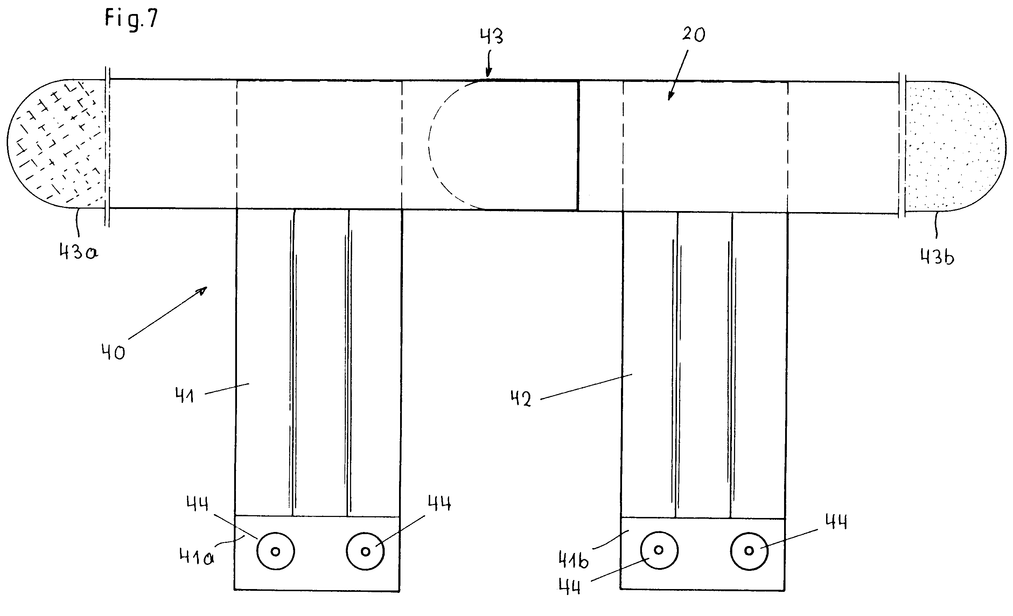

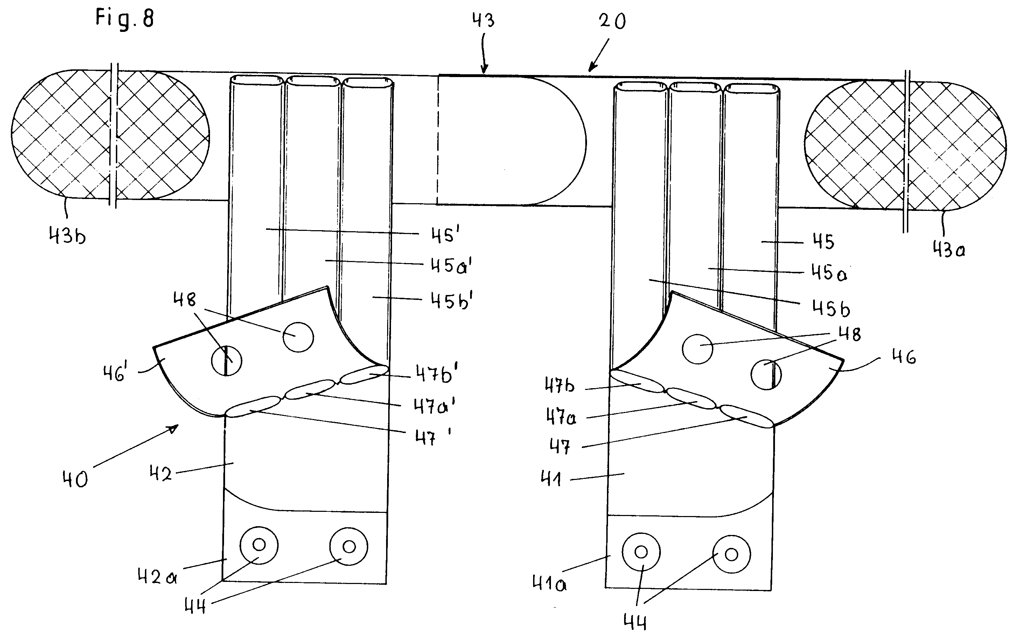

Die Abpolsterung des Knieschienenrahmens 10 erfolgt mittels zweier, auf die Rahmenteile 11,111 aufschiebbarer Polsterzuschnitte 40,140, von denen jeder Polsterzuschnitt 40 bzw. 140 aus zwei streifenförmigen Seitenteilen 41,42 und 141,142 besteht, wobei die am Oberschenkel anliegenden Seitenteile 41,42 des Polsterzuschnittes 40 über einen oberen Steg 43 mit seitlich verlängerten, als Verschlußteile eines Verschlußbandes 20 ausgebildeten Abschnitten 43a,43b verbunden sind. Die freien Enden der seitlichen Abschnitte 43a,43b sind als Teile eines Klettverschlusses ausgebildet, um bei angelegter Knieschiene den Verschluß herzustellen. Der die beiden Seitenteile 41,42 des Polsterzuschnittes 40 verbindende Steg 43 ist bevorzugterweise zweiteilig ausgebildet, wobei beide Teile in ihrem Überlappungsbereich mit den Teilen eines Klettverschlusses versehen sind, um beide Teile lösbar verbinden zu können, wobei durch diese lösbare Verbindung eine Längenveränderbarkeit des Steges 43 des Polsterzuschnittes 40 möglich ist, um in Anpassung an die Weite des oberen Rahmenteils 11 auch den Polsterzuschnitt 40 anpassen zu können (Fig. 7 und 8).

An den freien Enden der beiden Seitenteile 41,42 des Polsterzuschnittes 40 sind Laschen 41a,42a angeformt, die Verschlußmittel 44 aufweisen, die beispielsweise als Druckknopfverschluß ausgebildet sind. Bei dem Polsterzuschnitt 40 verlaufen seine beiden Seitenteile 41,42 etwa parallel zueinander. Die Länge der Seitenteile 41,42 des Polsterzuschnittes 40 entspricht in etwa der Länge der am oberen Rahmenteil 11 angeordneten Fixierungsstäbe 16,16a,16b und 16',16a',16b', worauf nachstehend noch näher eingegangen wird.

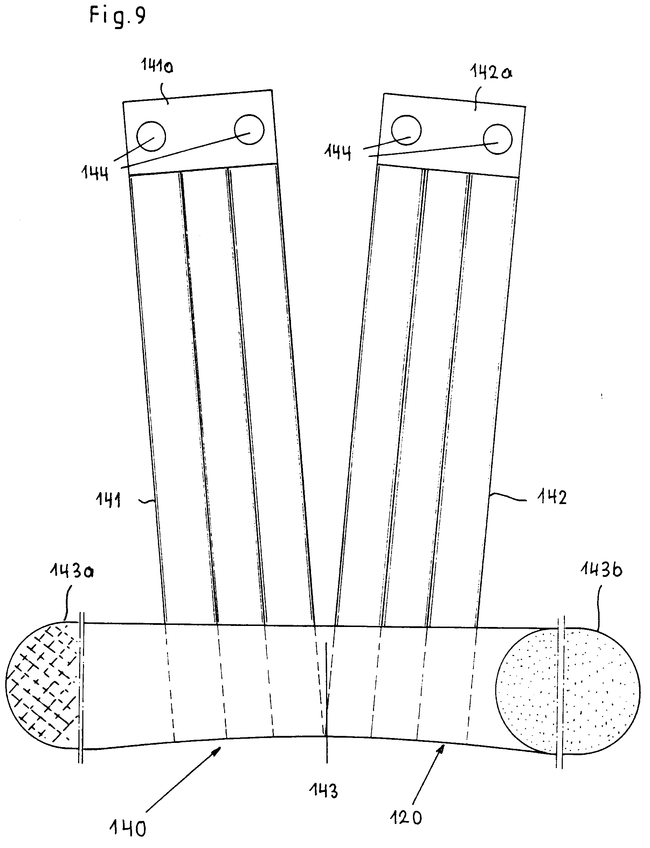

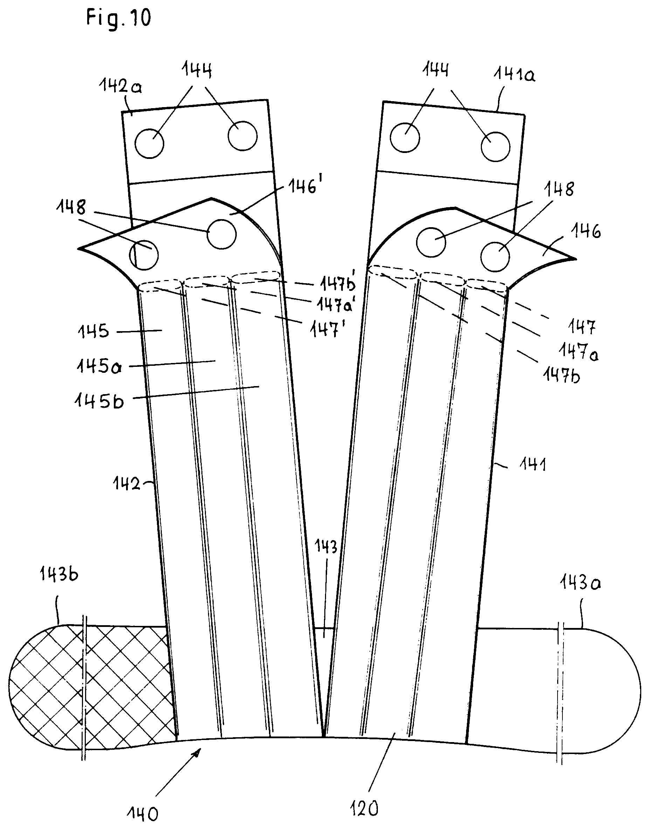

Der auf den unteren Rahmenteil 111 des Knieschienenrahmens 10 aufgeschobene Polsterzuschnitt 140 ist in etwa entsprechend dem Polsterzuschnitt 40 ausgebildet. Auch bei dem Polsterzuschnitt 140 sind die beiden Seitenteile 141,142 über einen Kurzsteg 143 einendseitig miteinander verbunden. In Verlängerung des Kurzsteges 143 sind an den Seitenteilen 141,142 seitliche Abschnitte 143a,143b angeformt, die an ihren Enden mit den Teilen eines Klettverschlusses versehen sind und im angelegten Zustand der Knieschiene 100 das untere Verschlußband 120 bilden. Die beiden Seitenteile 141,142 des Polsterzuschnitts 140 tragen Laschen 141a,142a, an denen Verschlußmittel 144, beispielsweise Druckknopfverschlüsse, angebracht sind (Fig.9 und 10).

Auch im Falle des Polsterzuschnittes 140 ist eine Anpassung an das untere Rahmenteil 111 des Knieschienenrahmens 10 insofern gegeben, als die beiden Seitenteile 141,142 des Polsterzuschnittes 140 leicht V-förmig ausgestellt sind, was in etwa dem anatomischen Verlauf der Wade des Unterschenkels entspricht.

Die dem Bein zugekehrten Flächen der beiden Polsterzuschnitte 40,140 bestehen bevorzugterweise aus einem Grundgewebe mit einer aufgebrachten Schicht aus einem geeigneten Polstermaterial, wobei Polstermaterialien aus natürlichen und auch aus künstlichen Fasern eingesetzt werden können. Auch federnd-elastische Kunststoffformkörper können in das Material der Polsterzuschnitte 40,140 eingearbeitet sein.

Um in die Polsterzuschnitte 40,140 die Fixierungsstäbe an den Rahmenteilen 11,111 des Knieschienenrahmens 10 aufschieben zu können, sind die Polsterzuschnitte rückseitig mit einer Anzahl von Taschen versehen, die einendseitig in Einschuböffnungen münden. Die Anzahl der Taschen zur Aufnahme der Fixierungsstäbe 16,16a,16b und 16',16a',16b' bzw. 116,116a,116b und 116',116a',116b' richtet sich nach der Anzahl der Fixierungsstäbe. Bei dem vorangehend beschriebenen und in der Zeichnung dargestellten Ausführungsbeispiel sind an dem oberen Rahmenteil 11 und an dem unteren Rahmenteil 111 jeweils sechs Fixierungsstäbe vorgesehen, von denen jeweils drei Fixierungsstäbe zu einer Einheit zusammengefaßt sind. Die Polsterzuschnitte 40,140 weisen hiernach auf ihren Rückseiten eine der Anzahl dieser Fixierungsstäbe entsprechende Anzahl von Taschen auf.

Bei dem Polsterzuschnitt 40 sind für die Fixierungsstäbe des oberen Rahmenteils 11 des Knieschienenrahmens 10 die Taschen 45,45a,45b und 45',45a',45b' mit den Einschuböffnungen 47,47a,47b und 47',47a',47b' vorgesehen.

Zur Aufnahme der Fixierungsstäbe an dem unteren Rahmenteil 111 des Knieschienenrahmens 10 weist der Polsterzuschnitt 140 auf seiner Rückseite die Taschen 145,145a, 145b und 145',145a',145b' mit den Einschuböffnungen 147, 147a,147b und 147',147a',147b' auf (Fig. 8 und 10). Die Länge der Taschen an den Rückseiten der Polsterzuschnitte 40,140 entspricht in etwa der Länge der aufzunehmenden Fixierungsstäbe an dem oberen Rahmenteil 11 und dem unteren Rahmenteil 111 des Knieschienenrahmens 10. Benachbart zu den Laschen 41a,42a der Seitenteile 41,42 des Polsterzuschnittes 40 und der Laschen 141a,142a an den Seitenteilen 141,142 des Polsterzuschnittes 140 sind auf der Rückseite der Polsterzuschnitte 40,140 Laschen 46,46' und 146,146' vorgesehen, die mit Durchbrechungen 48,148 versehen sind, um diejenigen Teile der Druckknopfverbindung, die an den Laschen 41a,42a und 141a,142a der Polsterzuschnitte 40,140 vorgesehen sind, in die entsprechenden Gegenteile der Druckknopfverschlüsse einführen zu können, wobei letztgenannte Druckknopfteile an den Außenseiten der halbkreisförmig verlaufenden Stege 13,113 der U-förmigen Stabprofile 12,112 befestigt sind. Hinsichtlich der Befestigungsmittel können anstelle von Druckknopfverschlüssen auch andere Befestigungsmittel vorgesehen sein; wesentlich ist, daß die Polsterzuschnitte 40,140 an den Rahmenteilen 11, 111 lösbar befestigt sind.

Die Ausbildung der Taschen 45,45a,45b und 45',45a',45b' bzw. 145,145a,145b und 145',145a',145b' der beiden Polsterzuschnitte 40,140 erfolgt durch eine doppelwandige Ausgestaltung des Grundgewebezuschnitts für die Polsterzuschnitte 40,140, wobei dann die Taschenausbildung durch Nähverbindungen hergestellt wird.

Die Befestigung der beiden Polsterzuschnitte 40,140 an den beiden Rahmenteilen 11,111 des Knieschienenrahmens 10 erfolgt in der Weise, daß die Laschen 41a,42a und 141a,142a der beiden Polsterzuschnitte 40,140 um die halbkreisförmig verlaufenden Stege 13,113 herumgelegt und die beiden Druckknopfverschlußteile in Wirkverbindung gebracht werden, nachdem beide Polsterzuschnitte 40,140 über die Fixierungsstäbe an den beiden Rahmenteilen 11, 111 geschoben worden sind.

Das jeweils obere und untere Verschlußband 20,120 der Knieschiene 100 ist an dem Polsterzuschnitt 40 bzw. an dem Polsterzuschnitt 140 befestigt. Die beiden weiteren Verschlußbänder 30,130, die oberhalb und unterhalb des Knies zu liegen kommen, sind dagegen an den beiden Rahmenteilen 11,111 des Knieschienenrahmens 10 befestigt. Auch hier können die Verschlußbänder von Laschen gebildet werden, die klettenverschlußartige Verbindungsmittel aufweisen. Vorteilhafter ist es jedoch, wenn die beiden Verschlußbänder 30,130 als Gurtbänder ausgebildet sind. Hierzu besteht jedes Verschlußband 30,130 aus einem bandförmigen Abschnitt, der durch eine auf der gegenüberliegenden Seite an den Rahmenteilen 11,111 befestigte Öse hindurchgeführt ist. Das freie Ende eines jeden Verschlußbandes wird nach Hindurchführen durch diesen Ring dann auf dem jeweils darunterliegenden Verschlußbandabschnitt vermittels eines Klettverschlusses befestigt.

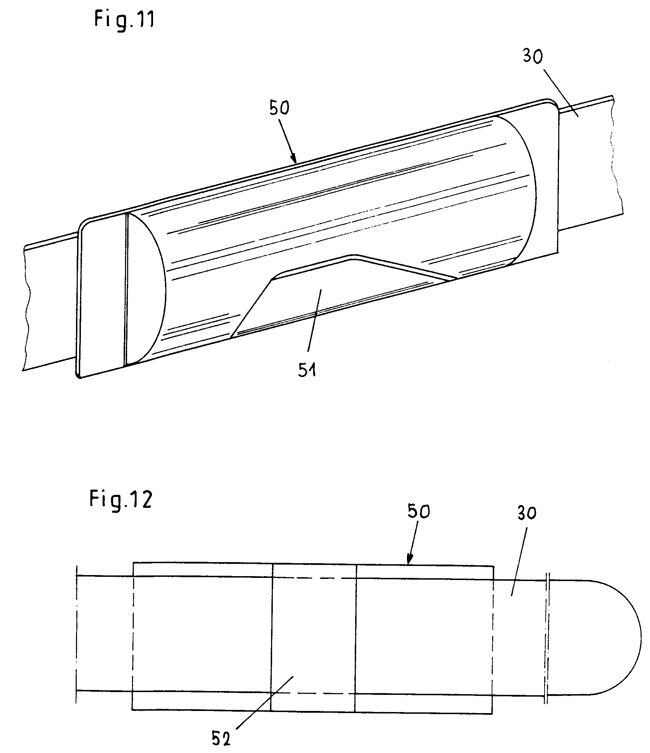

Das im unteren Bereich des oberen Rahmenteils 11 des Knieschienenrahmens 10 vorgesehene Verschlußband 30 trägt ein Polster bzw. Kissen 50 aus einem federnd-elastischen Material, wie z.B. einem Silikonkautschuk, oder einem anderen geeigneten Polstermaterial, wobei das Polster 50 mit einer der Patella angepaßten Formgebung versehen ist, die bei 51 in Fig.11 angedeutet ist. Das Polster 50 ist an dem Verschlußband 30 fest, lösbar und/oder verschiebbar gehalten. Für letztere Ausgestaltung ist das Polster 50 an seiner Rückseite mit einer Befestigungsschlaufe 52 versehen (Fig.12). Auch an dem unterhalb des Knies verlaufenden Verschlußband 130 kann zur Abstützung des Knies ein Polster bzw. Kissen 150 vorgesehen sein.

Die Stege 13,113 der Rahmenteile 11,111 können zusätzlich mit Polstern versehen sein.

Weitere Polsterungen können an den halbkreisförmig verlaufenden Stegen 13,113 der Stabprofile 12,112 des Rahmens 10 vorgesehen sein, wie dies in Fig.6 bei 60,160 angedeutet ist.

## Patentansprüche

1. Knieschiene mit einer am Bein anzulegenden, auf der Vorderseite des Beines mittels Verschlußbänder zusammengehaltenen Hülse aus einem abgepolsterten Rahmenteil, aus einem metallischen Rahmen (10) aus einem oberen, am Oberschenkel des Beines dorsal anlegbaren kürzeren, halbschalenförmigen Rahmenteil (11) und einem unteren, am Unterschenkel des Beines dorsal anlegbaren längeren, halbschalenförmigen Rahmenteil (111), wobei beide Rahmenteile (11; 111) miteinander verbunden sind und jeder Rahmenteil (11; 111) von einem U-förmigen Stabprofil (12; 112) mit einem etwa halbkreisförmig verlaufenden Steg (13; 113) zur rückwärtigen Anlage an den Oberschenkel bzw. den Unterschenkel des Beines gebildet ist, an dem seitlich und benachbart zu den beiden Stabprofilschenkeln (14, 15; 114, 115) eine Anzahl von einendseitig befestigten, in Längsrichtung der Knieschiene (100) zu dessen beiden Enden verlaufenden, verformbaren, federnden und eine hohe Eigensteifigkeit aufweisen, einarmige Fixierungsstäben (16, 16a, 16b; 16', 16a', 16b'; 116, 116a, 116b; 116', 116a', 116b') befestigt sind, die der anatomischen Form von Oberschenkel und Unterschenkel des Beines angepaßt sind, wobei die beiden Rahmenteile (11; 111) derart miteinander verbunden sind, daß deren beiden U-förmigen Stabprofile (12; 112) einander zugekehrt sind, und wobei die Knieschiene (100) im Bereich ihrer beiden Endbereiche die Knieschiene (100) mit je einem Verschlußband (20; 120) und oberhalb und unterhalb des Knies mit je einem weiteren Verschlußband (30; 130) versehen ist.

2. Knieschiene nach Anspruch 1,
dadurch gekennzeichnet,
daß die beiden Rahmenteile (11; 111) des Knieschienenrahmens (10) über die freien Enden der Schenkel (14, 15; 114, 115) der beiden U-förmigen Stabprofile (12; 112) gelenkig miteinander verbunden sind, wobei die Gelenke (18; 118) mit einer Verstelleinrichtung zum Arretieren der beiden Rahmenteile (11; 111) in einer vorgegebenen Winkelstellung oder zum Einstellen von dem Genesungszustand des Beines anpaßbaren und veränderbaren Winkelbereichen für eine definierte Beinbewegung zwischen zwei in ihren Endpunkten festgelegten Winkelbereichen versehen sind.

3. Knieschiene nach einem der Ansprüche 1 und 2,
dadurch gekennzeichnet,
daß der halbkreisförmig verlaufende Steg (13; 113) eines jeden Rahmenteils (11; 111) aus zwei längenveränderbaren Stababschnitten (13a, 13b; 113a, 113b) besteht, die mittels einer Feststelleinrichtung (17; 117) bevorzugterweise mittels einer Schraubverbindung feststellbar sind.

4. Knieschiene nach einem der Ansprüche 1 bis 3,
dadurch gekennzeichnet,
daß die die beiden Rahmenteile (11; 111) des Knieschienenrahmens (10) miteinander verbindenden Gelenke (18; 118) derart zueinander angeordnet sind,
daß deren Schwenkachsen (18a; 118a) kongruent zueinander verlaufen.

5. Knieschiene nach einem der Ansprüche 1 bis 4,
dadurch gekennzeichnet,
daß die U-förmigen Stabprofile (12; 112) der beiden Rahmenteile (11; 111) aus einem Flach- bzw. Bandprofil bestehen, wobei die die halbkreisförmig verlaufenden Stege (13; 113) und die Stabprofilschenkel (14, 15; 114, 115) bildenden Abschnitte senkrecht aufeinander und um 90° zueinander versetzt sind.

6. Knieschiene nach einem der Ansprüche 1 bis 5,
dadurch gekennzeichnet,
daß jedes Rahmenteil (11; 111) des Knieschienenrahmens (10) je drei dorsale Fixierungsstäbe (16, 16a, 16b; 16', 16a', 16b'; 116, 116a, 116b; 116', 116a', 116b') aufweist, die in etwa parallel zueinander verlaufen.

7. Knieschiene nach einem der Ansprüche 1 bis 6,
dadurch gekennzeichnet,
daß die Fixierungsstäbe (116, 116a, 116b; 116', 116a', 116b') des unteren Rahmenteils (111) des Knieschienenrahmens (10) gerade verlaufend mit Abwinkelungen im unteren Wadenbereich des Beines ausgebildet sind.

8. Knieschiene nach einem der Ansprüche 1 bis 7,
dadurch gekennzeichnet,
daß die Fixierungsstäbe (16, 16a, 16b; 16', 16a', 16b'; 116, 116a, 116b; 116', 116a', 116b') rohrförmig ausgebildet sind und einen kreisförmigen Querschnitt aufweisen.

9. Knieschiene nach einem der Ansprüche 1 bis 8,
dadurch gekennzeichnet,
daß die Fixierungsstäbe (16, 16a, 16b; 16', 16a', 16b'; 116, 116a, 116b; 116', 116a', 116b') aus einem Leichtmetall, zweckmäßigerweise aus Aluminium, glasfaserverstärktem Kunststoff bestehen oder als Kohlefaserstäbe ausgebildet sind.

10. Knieschiene nach einem der Ansprüche 1 bis 9,
dadurch gekennzeichnet,
daß die Fixierungsstäbe (16, 16a, 16b; 16', 16a', 16b'; 116, 116a, 116b; 116', 116a', 116b') vollwandig ausgebildet sind.

11. Knieschiene nach einem der Ansprüche 1 bis 10,
dadurch gekennzeichnet,
daß zur Abpolsterung des Knieschienenrahmens (10) zwei auf die Fixierungsstäbe (16, 16a, 16b; 16', 16a', 16b'; 116, 116a, 116b; 116', 116a', 116b') der beiden Rahmenteile (11; 111) des Knieschienenrahmens (10) aufschiebbare Polsterzuschnitte (40; 140) vorgesehen sind, von denen jeder Polsterzuschnitt (40; 140) von zwei streifenförmigen Seitenteilen (41, 42; 141, 142) gebildet ist, von denen die am Oberschenkel des Beines anliegenden Seitenteile (41, 42) des Polsterzuschnittes (40) über einen oberen Steg (43) mit seitlich verlängerten als Verschlußteile eines Verschlußbandes (20) ausgebildeten Abschnitten (43a, 43b) verbunden sind, wobei die am Unterschenkel des Beines anliegenden Seitenteile (141, 142) des Polsterzuschnittes (140) über einen unteren Steg (143) mit seitlich verlängerten als Verschlußteile eines Verschlußbandes (120) ausgebildeten Abschnitten (143a, 143b) verbunden sind, und daß die freien Enden der Seitenteile (41, 42; 141, 142) der beiden Polsterzuschnitte (40; 140) Laschen (41a, 42a; 141a, 142a) mit Verschlußmitteln (44; 144) wie bevorzugterweise Druckknöpfe, aufweisen, vermittels der die Polsterzuschnitte (40; 140) an den halbkreisförmig verlaufenden Stegen (13; 113) der U-förmigen Stabprofile (12; 112) der beiden Rahmenteile (11; 111) anschließbar bzw. befestigbar sind, wobei die Rückseiten der Seitenteile (41, 42; 141, 142) der beiden Polsterzuschnitte (40; 140) mit einer der Anzahl der Fixierungsstäbe (16, 16a, 16b; 16', 16a', 16b'; 116, 116a, 116b; 116', 116a', 116b') der Rahmenteile (11; 111) entsprechende Anzahl von Taschen (45, 45a, 45b; 45', 45a', 45b'; 145, 145a, 145b; 145', 145a', 145b') mit Einschuböffnungen (47, 47a, 47b; 47', 47a', 47b'; 147, 147a, 147b; 147', 147a', 147b') für die Fixierungsstäbe sowie mit je einer Lasche (46, 46'; 146, 146') mit Durchbrechungen (48; 148) zum Hindurchführen der Verschlußmittel (44; 144) versehen sind.

12. Knieschiene nach einem der Ansprüche 1 bis 11,
dadurch gekennzeichnet,
daß von den vier Verschlußbändern (20, 30; 120, 130), die an den Enden der Knieschiene (100) vorgesehenen Verschlußbänder (20; 120) an den Polsterzuschnitten (40; 140) und die beiden anderen Verschlußbänder (30; 130) sich gegenüberliegend oberhalb und unterhalb des Kniebereiches an dem Knieschienenrahmen (10) benachbart zu den Gelenkschwenkachsen (18a; 118a) der beiden Rahmenteile (11; 111) befestigt sind.

13. Knieschiene nach einem der Ansprüche 1 bis 12,
dadurch gekennzeichnet,
daß die die Verschlußbänder (20; 120) bildenden Bandabschnitte (43a, 43b; 143a, 143b) an den Polsterzuschnitten (40; 140) an ihren freien Enden mit den Klettverschlüsse bildenden Teilen versehen sind.

14. Knieschiene nach einem der Ansprüche 1 bis 13,
dadurch gekennzeichnet,
daß das an dem oberen Rahmenteil (11) des Knieschienenrahmens (10) befestigte und oberhalb des Knies bei angelegter Knieschiene (100) verlaufende Verschlußband (30) ein Polster (50) trägt, das aus einem federnd-elastischen Material oder einem anderen geeigneten Polstermaterial besteht und mit einer der Patella angepaßten Formgebung (51) versehen ist, wobei das Polster (50) an dem Verschlußband (30) fest, lösbar oder verschiebbar gehalten ist.

## Claims

1. Knee splint with a sleeve to be applied to the leg, held together at the front of the leg with the aid of closing straps, comprised of a padded frame portion, of a metallic frame (10) a shorter, half shell-shaped frame portion (11) dorsally applicable to the thigh and of a lower, longer, half shell-shaped frame portion (111) dorsally applicable to the lower leg, in which case both frame portions (11 ; 111) are interconnected and each frame portion (11 ; 111) is formed by a U-shaped rod section (12 ; 112) with an approximately semicircularly proceeding web (13 ; 113) for rearwardly bearing against the thigh or the lower leg, on which, laterally and adjacent to the two rod section legs (14, 15; 114, 115), a plurality of one-armed fixing rods (16, 16a, 16b; 16', 16a', 16b'; 116, 116a, 116b; 116', 116a', 116b'), attached at one end and proceeding in the longitudinal direction of the knee splint (100) relative to the two ends of the same, said rods being deformable, springable and possessing a high degree of inherent rigidity and being adapted to the anatomical configuration of thigh and lower leg, while the two frame portions (11 ; 111) are interconnected in such a way that their two U-shaped rod sections are facing each other and the knee splint, within the area of its two terminal regions, the knee splint (100) is provided with one closing strap (20; 120) each and, above and below the knee, with one further closing strap (30 ; 130) each.

2. Knee splint according to Claim 1,
characterized in that
the two frame portions (11 ; 111) of th knee splint frame (10), via the free ends of the legs (14, 15; 114, 115) of the two U-shaped rod sections (12 ; 112) are hingedly interconnected, while the articulations (18 ; 118) are provided with an adjacent means for locking the two frame portions (11 ; 111) in a predetermined angular position or for the adjustment of, from the recovery state of the leg, adaptable and changeable angular ranges for a defined motion of the leg between two angular ranges that are fixed between their terminal points.

3. Knee splint according to either Claim 1 or 2,
characterized in that
the semicircularly proceeding web (13 ; 113) of each frame portion (11 ; 111) is comprised of two longitudinally variable rod sections (13a, 13b ; 113a, 113b), which, with the aid of an immobilizing device (17 ; 117), by preference with the aid of a screwed connection, can be immobilized.

4. Knee splint according to any of Claims 1 to 3,
characterized in that
the articulations (18 ; 118) interconnecting the two frame portions (11 ; 111) of the knee splint frame (10) are disposed relative to each other in such a way that their swivel axes (18a ; 118a) proceed congruently to each other.

5. Knee splint according to any of Claims 1 to 4,
characterized in that
the U-shaped rod sections (12 ; 112) of the two frame portions (11 ; 111) are comprised of a flat or band-shaped section, in which case the semi circularly proceeding webs (13 ; 113) and the sections constituting the rod section legs (14, 15 ; 114, 115) are offset vertically upon each other and relative to each other by 90°.

6. Knee splint according to any of Claims 1 to 5,
characterized in that
each frame portion (11 ; 111) of the knee splint frame (10) possesses three dorsal fixation rods each (16, 16a, 16b ; 16', 16a', 16b' 116', 116a', 116b'), which proceed approximately parallel to each other.

7. Knee splint according to any of Claims 1 to 6,
characterized in that
the fixing rods (116, 116a, 116b ; 116', 116a', 116b') of the lower frame portion (111) of the knee splint frame (10) are constructed in so as to proceed straight with angled sections within the lower calf region of the leg.

8. Knee splint according to any of Claims 1 to 7,
characterized in that
the fixing rods (16, 16a, 16b ; 16', 16a', 16b' ; 116, 116a, 116b ; 116', 116a', 116b' ) are of tubular construction and possess a circular cross section.

9. Knee splint according to any of Claims 1 to 8,
characterized in that
the fixing rods (16, 16a, 16b ; 16', 16a', 16b' ; 116, 116a, 116b ; 116', 116a', 116b') are comprised of a light alloy, expediently of aluminium, glass fiber reinforced plastic or in the form of carbon fiber rods.

10. Knee splint according to any of Claims 1 to 9,
characterized in that
the fixing rods (16, 16a, 16b ; 16', 16a', 16b' ; 116, 116a, 116b ; 116', 116a', 116b') are of solid construction.

11. Knee splint according to any of Claims 1 to 10,
characterized in that,
for padding the knee splint frame (10), two padding blanks (40 ; 140) that can be slid onto the fixing rods (16, 16a, 16b ; 16', 16a', 16b' ; 116, 116a, 116b ; 116', 116a', 116b') of both frame portions (11 ; 111) of the knee splint frame (10) are provided, of which each padding blank (40 ; 140) is formed by two strip-like side portions (41, 42 ; 141, 142), of which the side portions (41, 42) that fit closely to the thigh of the padding blank (40) is, via an upper web (43), connected with laterally extended sections (43a, 43b) constructed in the form of closing elements of a closing strap (20), while the side portions (141, 142) fitting closely to the lower leg of the padding blank (140), via a lower web (143), are connected with laterally extended sections (143, 143b) constructed in the form of closing elements of a closing strap (120), and in that the free ends of the side portions (41, 42; 141, 142) of the two podding blanks (40 ; 140) are provided with tabs (41a, 42b ; 141a, 142b) with closing elements (44, 144), such as by preference press studs, with the aid of which the padding blanks (40 ; 140) are connect able or securable to the semicircularly extending webs (13 ; 113) of the U-shaped rod sections (12 ; 112) of the two frame portions (11 ; 111), in which case the rears of the lateral portions (41, 42 ; 141, 142) of the two padding blanks (40 ; 140) are provided with a number of pockets (45, 45a, 45b ; 45', 45a', 45b'; 145, 145a, 145b ; 145', 145a', 145b') corresponding to the number of the fixing rods (16, 16a, 16b; 16', 16a', 16b' ; 116, 116a, 116b;, 116', 116a', 116b') of the frame portions (11 ; 111) with insertion apertures (47, 47a, 47b ; 47', 47a', 47b' ; 147, 147a, 147b ; 147' 147a', 147b') for the fixing rods as well as with one tab each (46, 46' ; 146, 146') with perforations (48 ; 148) for passing the closing elements through.

12. Knee splint according to any of Claims 1 to 11,
characterized in that,
of the four closing straps (20, 30 ; 120, 130), the closing straps (20 ; 120) provided on the ends of the knee splint (100) are attached to the padding blanks (40 ; 140) and the other two closing straps (30 ; 130) are attached so as to be located opposite each other above and below the knee area on the knee splint frame (10) adjacent to the articulation swivel axes (18a ; 118a) of the two frame portions (11 ; 111).

13. Knee splint according to any of Claims 1 to 12,
characterized in that
the strap sections (43a, 43b ; 143a, 143b) forming the closing straps (20 ; 120) on the padding blanks (40 ; 140), on their free ends, are provided with the portions that constitute the Velcro fasteners.

14. Knee splint according to any of Claims 1 to 13,
characterized in that
the closing strap (30) attached to the upper frame portion (11) of the knee splint frame (10) when the knee splint (100) is applied, while proceeding above the knee, carries a padding (50>) that is comprised of a springably-elastic material or some other suitable padding material, the padding (50) being retained irremovably, detachably or displaceably on the closing strap (30).

## Revendications

1. Eclisse de genou avec une gaine à appliquer sur la jambe, maintenue sur la face avant de la jambe au moyen de bandes de fermeture, constituée par une partie de cadre rembourrée, par un cadre métallique (10) constitué par une partie de cadre supérieure (11) en forme de demi-coque plus courte devant être appliquée dorsalement sur la cuisse de la jambe et une partie de cadre inférieure en forme de demi-coque plus longue (111), devant être appliquée dorsalement sur la partie inférieure de la jambe, les deux parties de cadre (11 ; 111) étant reliées l'une à l'autre et chaque partie de cadre (11 ; 111) étant formée par une baguette profilée en forme d'U (12 ; 112) avec une entretoise (13 ; 113) allant à peu près en forme de demi-cercle pour l'appui arrière sur la cuisse ou sur la partie inférieure de la jambe, entretoise sur laquelle sont fixées un certain nombre de baguettes de fixation à un bras (16, 16a, 16b ; 16', 16a', 16b' ; 116, 116a, 116b ; 116', 116a', 116b'), déformables, élastiques et présentant une grande rigidité propre, fixées du côté d'une extrémité, allant dans le sens longitudinal de l'éclisse de genou (100) vers les deux extrémités de celle-ci, baguettes qui sont adaptées à la forme anatomique de la cuisse et de la partie inférieure de la jambe, les deux parties de cadre (11 ; 111) étant reliées l'une à l'autre, que leurs deux baguettes profilées en forme d'U (12 ; 112) sont tournées l'une vers l'autre, et l'éclisse de genou (100) étant pourvue, dans la zone de chacune de ses deux zones d'extrémité, d'une bande de fermeture (20 ; 120) et de respectivement une autre bande de fermeture (30 ; 130) au-dessus et au-dessous du genou.

2. Eclisse de genou selon la revendication 1,
caractérisée en ce
que les deux parties de cadre (11 ; 111) du cadre de l'éclisse de genou (10) sont reliées l'une à l'autre de manière articulée par les extrémités libres des montants (14, 15 ; 114, 115) des deux baguettes profilées en forme d'U (12 ; 112), les articulations (18 ; 118) étant pourvues d'un dispositif de réglage pour arrêter les deux parties de cadre (11 ; 111) dans une position angulaire prédéfinie ou pour régler des zones angulaires variables et pouvant être adaptées à l'état de rétablissement de la jambe pour un mouvement défini de la jambe entre deux zones angulaires fixées dans leurs points d'extrémité.

3. Eclisse de genou selon l'une des revendications 1 et 2,
caractérisée en ce
que la baguette en forme de demi-cercle (13 ; 113) de chacune des parties de cadre (11 ; 111) est constituée par deux portions de baguette de longueur réglable (13a, 13b ; 113a, 113b) qui peuvent être bloquées au moyen d'un dispositif de biocage (17 ; 117) de préférence au moyen d'un assemblage à vis.

4. Eclisse de genou selon l'une des revendications 1 à 3,
caractérisée en ce
que les articulations (18 ; 118) qui relient l'une à l'autre les deux parties de cadre (11 ; 111) du cadre de l'éclisse de genou (10) sont disposées l'une par rapport à l'autre de telle manière que leurs axes de pivotement (18a ; 118a) sont congrus l'un vers l'autre.

5. Eclisse de genou selon l'une des revendications 1 à 4,
caractérisée en ce
que les baguettes profilées en forme d'U (12 ; 112) des deux parties de cadre (11 ; 111) sont constituées par un profilé plat ou un profilé en bande, les sections qui forment les entretoises en forme de demi-cercle (13 ; 113) et les montants de baguette profilée (14, 15 ; 114, 115) étant perpendiculaires l'un à l'autre et décalés de 90° l'un par rapport à l'autre.

6. Eclisse de genou selon l'une des revendications 1 à 5,
caractérisée en ce
que chaque partie de cadre (11 ; 111) du cadre de l'éclisse de genou (10) présente trois baguettes de fixation dorsales (16, 16a, 16b ; 16', 16a', 16b' ; 116, 116a, 116b ; 116', 116a', 116b') chacune qui sont à peu près parallèles l'une à l'autre.

7. Eclisse de genou selon l'une des revendications 1 à 6,
caractérisée en ce
que les baguettes de fixation (116, 116a, 116b ; 116', 116a', 116b') de la partie de cadre inférieure (111) du cadre de l'éclisse de genou (10) sont configurées droites avec des coudures dans la zone inférieure du mollet de la jambe.

8. Eclisse de genou selon l'une des revendications 1 à 7,
caractérisée en ce
que les baguettes de fixation (16, 16a, 16b ; 16', 16a', 16b' ; 116, 116a, 116b ; 116', 116a', 116b') sont configurées en forme de tube et présentent une section circulaire.

9. Eclisse de genou selon l'une des revendications 1 à 8,
caractérisée en ce
que les baguettes de fixation (16, 16a, 16b ; 16', 16a', 16b' ; 116, 116a, 116b ; 116', 116a', 116b') sont en un métal léger, de manière appropriée en aluminium, en matière plastique renforcée par des fibres de verre ou sont configurées comme des baguettes en fibres de carbone.

10. Eclisse de genou selon l'une des revendications 1 à 9,
caractérisée en ce
que les baguettes de fixation (16, 16a, 16b ; 16', 16a', 16b' ; 116, 116a, 116b ; 116', 116a', 116b') sont configurées à parois pleines.

11. Eclisse de genou selon l'une des revendications 1 à 10,
caractérisée en ce
que, pour rembourrer le cadre de l'éclisse de genou (10), deux découpes de rembourrage (40 ; 10) enfilables sur les baguettes de fixation (16, 16a, 16b ; 16', 16a', 16b' ; 116, 116a, 116b ; 116', 116a', 116b') des deux parties de cadre (11 ; 111) du cadre de l'éclisse de genou (10) sont prévues, découpes de rembourrage dont chacune (40 ; 140) est formée par deux parties latérales en forme de bande (41, 42 ; 141, 142) parmi lesquelles les parties latérales (41, 42) de la découpe de rembourrage (140) qui adhèrent à la cuisse de la jambe sont reliées par une entretoise supérieure (43) aux sections (43a, 43b) prolongées latéralement, configurées comme pièces de fermeture d'une bande de fermeture (20), les parties latérales (141) 142) de la découpe de rembourrage (140) qui adhèrent à la partie inférieure de la jambe étant reliées par une entretoise inférieure (143) aux sections (143a, 143b) prolongées latéralement, configurées comme pièces de feremture d'une bande de fermeture (120), et que les extrémités libres des parties latérales (41, 42 ; 141, 142) des deux découpes de rembourrage (40 ; 140) présentent des attaches (41a, 42a ; 141a, 142a) avec des moyens de fermeture (44 ; 144) comme de préférence des boutons de pression au moyen desquels les découpes de rembourrage (40 ; 140) peuvent être raccordées ou fixées aux entretoises en forme de demi-cercle (13 ; 113) des baguettes profilées en forme d'U (12 ; 112) des deux parties de cadre (11 ; 111), les faces arrières des parties latérales (41, 42 ; 141, 142) des deux découpes de rembourrage (40 ; 140) étant pourvues d'un nombre de poches (45, 45a, 45b ; 45', 45a', 45b' ; 145, 145a, 145b ; 145', 145a', 145b') avec des ouvertures d'insertion (47, 47a, 47b ; 47', 47a', 47b' ; 147, 147a, 147b ; 147', 147a', 147b') pour les baguettes de fixation, nombre qui correspond au nombre des baguettes de fixation (16, 16a, 16b ;16', 16a', 16b' ; 116, 116a, 116b ; 116', 116a', 116b') des parties de cadre (11 ; 111) ainsi qu'avec respectivement une attache (46, 46' ; 146, 146') avec des découpures (48 ; 148) pour faire passer les moyens de fermeture (44 ; 144).

12. Eclisse de genou selon l'une des revendications 1 à 11,
caractérisée en ce
que, parmi les quatre bandes de fermeture (20, 30 ; 120, 130), les bandes de fermeture (20 ; 120) prévues aux extrémités de l'éclisse de genou (100) sont fixées aux découpes de rembourrage (40 ; 140) et les deux autres bandes de fermeture (30 ; 130) sont fixées en face au-dessus et au-dessous de la zone du genou sur le cadre de l' éclisse de genou (10) au voisinage des axes de pivotement des articulations (18a ; 118a) des deux parties de cadre (11 ; 111).

13. Eclisse de genou selon l'une des revendications 1 à 12,
caractérisée en ce
que les sections de bande (43a, 43b ; 143a, 143b) formant les bandes de fermeture (20 ; 120) sont pourvues sur les découpes de rembourrage (40 ; 140) à leurs extrémités libres de parties qui forment des fermetures auto-agrippantes.

14. Eclisse de genou selon l'une des revendications 1 à 13,
caractérisée en ce
que la bande de fermeture (30), fixée sur la partie de cadre supérieure (11) du cadre de l'éclisse de genou (10) et qui passe au-dessus du genou, lorsque l'éclisse de genou (100) est appliquée, porte un rembourrage (50) qui est constitué par une matière élastique à la manière d'un ressort ou une autre matière de rembourrage appropriée et qui est pourvu d'une conformation (51) adaptée à la rotule, le rembourrage (50) étant maintenu de manière fixe, amovible ou glissante sur la bande de fermeture (30).
